(19) Europäisches Patentamt
European Patent Office
Office européen des brevets

(11) EP 1 782 788 A1

(12) EUROPEAN PATENT APPLICATION
published in accordance with Art. 158(3) EPC

(43) Date of publication:
**09.05.2007 Bulletin 2007/19**

(21) Application number: **05758277.7**

(22) Date of filing: **08.07.2005**

(51) Int Cl.:
***A61K 6/00*** *(2006.01)*

(86) International application number:
**PCT/JP2005/012692**

(87) International publication number:
**WO 2006/006541 (19.01.2006 Gazette 2006/03)**

(84) Designated Contracting States:
**DE FR GB NL**

(30) Priority: **08.07.2004 JP 2004202137**

(71) Applicant: **KAO CORPORATION**
**Tokyo 103-8210 (JP)**

(72) Inventor: **HIRANO, Yuji,**
**c/o Kao Corp. Research Laboratories**
**Tokyo 1318501 (JP)**

(74) Representative: **HOFFMANN EITLE**
**Patent- und Rechtsanwälte**
**Arabellastrasse 4**
**81925 München (DE)**

# (54) REDUCING COMPOSITION FOR PERMANENT WAVE TREATMENT OR STRAIGHT PERMANENT TREATMENT

(57) The present invention relates to a hair reducing composition having the composition pH in a range of 8 to 10, containing (A) 0.039 to 0.39 mol/L of a keratin reducing substance not having a mercapto group selected from a sulfite, a hydrogen sulfite, or the like, (B) 0.007 to 0.196 mol/L of a keratin reducing substance having a mercapto group selected from a thioglycol acid, a cysteine, a salt thereof, or the like, (C) 0.1 to 10% by weight of a cationic surfactant and (D) 0.1 to 10% by weight of polyols;
and a permanent wave or straight permanent processing method of applying an oxidizing process with an oxidizing composition containing a keratin oxidizing substance after processing the hair with the above-mentioned reducing composition.



Printed by Jouve, 75001 PARIS (FR)

## Description

Field of the Invention

The present invention relates to a reducing composition for permanent wave or straight permanent, capable of providing a desired shape while restraining further damage even to drastically damaged hair by repeated chemical processes such as hair color, hair bleach, straight permanent and wave permanent, and a hair processing method using the same.

Background of the Invention

As a common means for setting hair in a desired shape over a long period, a wave permanent process and a straight permanent process can be presented. According to an ordinary permanent process, the stress generated at the time of deforming the hair is alleviated by the cleavage of the cysteine bond present inside the hair by the first agent containing a reducing substance such as a thioglycol acid salt and a cysteine. Then, the stress is restored by re-forming the cysteine bond by the second agent containing an oxidizing substance such as a bromate and a hydrogen peroxide for fixation in a desired shape. The shape is provided to the hair by winding the hair around a rod, or the like before and after the application of the first agent in the case of the wave permanent, and by straightly stretching by combing, or the like after the application of the first agent in the case of the straight permanent.

Reflecting the recent hair care circumstances, occasions of applying the permanent process to the hair drastically damaged by the repeatedhair color or hair bleach processes have increased. In the application of the permanent to such drastically damaged hair, even by the oxidization process after the reducing process with the first agent, the elasticity of the hair cannot be restored sufficiently. As aresult, with a remarkable loss of elasticity, not only is there decline of the permanent effect and sustenance, but the generation of hair breakage and deterioration of the feeling are also brought about.

In view of the background, for safely carrying out the permanent process to drastically damaged hair, many techniques have been disclosed. As one of them, several techniques utilizing a sulfite have been reported. For example, a technique for restraining the hair damage in the oxidization process without the need of the oxidization process with an oxidizing composition by use of one liquid type permanent wave agent containing a reducing agent such as a mercaptan compound, an air oxidization promoting agent and a sulfite compound, is known (see the patent document 1). Moreover, a technique of restraining the hair damage by controlling the effect and sustenance of the wave by processing the hair with a reducing lotion prepared by mixing a sulfite, or the like and a cysteine as the mercaptan compound by a specific ratio, and then having an intermediate process with a re-shaping lotion of pH 7 to 12 not including an oxidizing agent, and processing with a neutralizing lotion containing an oxidizing agent (patent document 2), and a technique of obtaining an excellent curl providing effect and glossiness by a hair processing composition including a sulfite or the like, and , for example, a cysteamine as the mercaptan compound (sulfhydryl compound) (patent document 3) are also known.

However, any of the methods is not sufficient in terms of providing a desired shape while restraining further damage to a minimum degree to the hair drastically damaged by the repeated chemical processes.

[Patent Document 1] JP-A-57-2216
[Patent Document 2] JP-A-63-190814
[Patent Document 3] JP-A-2-138112

Disclosure of the Invention

The present invention provides a reducing composition for permanent wave or straight permanent having the composition pH in a range of 8 to 10, containing the following components (A) to (D) :

(A) 0.039 to 0.39 mol/L of a keratin reducing substance not having a mercapto group selected from the group consisting of a sulfite, a hydrogen sulfite, a pyrosulfite and a thiosulfate,
(B) 0.007 to 0.196 mol/L of a keratin reducing substance having a mercapto group selected from the group consisting of a thioglycol acid and a salt thereof , a cysteine and a salt thereof , an acetyl cysteine, a cysteamine and a salt thereof, and a thiolactic acid and a salt thereof,
(C) 0.1 to 10% by weight of a cationic surfactant represented by the following general formula:

[chemical formula 1]

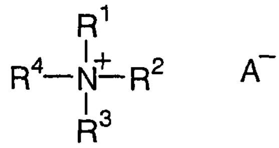

(wherein one or two of $R^1$, $R^2$, $R^3$ and $R^4$ is a hydrocarbon group having 12 to 30 carbon atoms, which may have a substituent, and the remainder are a hydrocarbon group having 1 to 3 carbon atoms, which may have a substituent, and $A^-$ is an anion), and

(D) 0.1 to 10% by weight of polyols.

Furthermore, the present invention provides a permanent wave or straight permanent processing method of processing hair with an oxidizing composition containing a keratin oxidizing substance selected from the group consisting of a bromate and a hydrogen peroxide after processing the hair with the above-mentioned reducing composition, washing with water or without washing.

Brief Description of the Drawings

FIG. 1 is a diagram showing the measurement kit used for the measurement of the elastic modulus of the hair in Example 1 and Comparative Examples 1 to 8.

Mode for Carrying out the Invention

The present invention relates to a reducing composition for permanent wave or straight permanent, capable of providing a desired shape while restraining further damage even to drastically damaged hair by repeated chemical processes such as hair color, hair bleach, straight permanent and wave permanent, and a hair processing method.

The present inventors have found out that a reducing composition satisfying the above-mentioned demands can be obtained by using a specific compound not having a mercapto group in combination with a specific compound having a mercapto group as a keratin reducing substance, and using a cationic surfactant and polyols.

The component (A) is a keratin reducing substance not having a mercapto group, selected from the group consisting of a sulfite, a hydrogen sulfite, a pyrosulfite and a thiosulfate. As the salts, a sodium salt, a potassium salt, an ammonium salt of organic amines (such as a monoethanolamine) can be presented. Among these examples, from the viewpoints of obtaining the preferable wave providing effect, curly hair correcting effect while restraining unpleasant smell and damage of the hair, it is preferable to use a sulfite and a hydrogen sulfite, and a sodium salt thereof is more preferable. These components (A) can be used by two or more kinds in combination, and the content thereof is preferably 0.039 to 0.39 mol/L in the reducing composition of the present invention, more preferably 0.039 to 0.31 mol/L, and even more preferably 0.039 to 0.28 mol/L from the viewpoints of the hair deforming effect (wave providing effect, curly hair correcting effect), safety, and bad-smell reduction at the time of use.

The component (B) is a keratin reducing substance having a mercapto group, selected from the group consisting of a thioglycol acid and a salt thereof, a cysteine and a salt thereof, an acetyl cysteine, a cysteamine and a salt thereof, and a thiolactic acid and a salt thereof. As the salts of a thioglycol acid and a thiolactic acid, an ammonium salt, and an ammonium salt of organic amines (such as a monoethanolamine), and as the salts of a cysteine and a cysteamine, a hydrochloride can be presented. Among these examples, from the viewpoints of obtaining the preferable hair deforming effect (wave providing effect, curly hair correcting effect), a thioglycol acid and' a salt thereof, a cysteine and a salt thereof, and an acetyl cysteine are preferable. These components (B) can be used by two or more kinds in combination, and the content thereof is preferably 0.007 to 0.196 mol/L in the reducing composition of the present invention, more preferably 0.007 to 0.163 mol/L, even more preferably 0.007 to 0.098 mol/L, and even more preferably 0.033 to 0.098 mol/L from the viewpoints of the hair deforming effect and safety.

The cationic surfactant of the component (C) is represented by the above-mentioned general formula. In the above-mentioned general formula, as the hydrocarbon group of $R^1$ to $R^4$, a straight chain or branched alkyl group, a straight chain or branched alkenyl group, an aryl group, an aralkyl group, or the like can be presented. As the substituent, a hydroxyl group, an alkoxy group, an aryloxy group, an epoxy group, an amino group, a mono- or dialkyl amino group, a trialkyl ammonium group, an aliphatic amide group, an aliphatic ester group, or the like can be presented. As an anion of $A^-$, a chloride ion, a bromide ion, a methylsulfuric acid ion, an ethyl sulfuric acid ion, a phosphoric acid ion, a sulfuric acid ion, a lactic acid ion, a saccharin ion, or the like can be presented.

As the specific examples of the cationic surfactant, a lauryl trimethyl ammonium chloride, a lauryl trimethyl ammonium bromide, a cetyl trimethyl ammonium chloride, a cetyl trimethyl ammonium bromide, a stearyl trimethyl ammonium chloride, a stearyl trimethyl ammonium bromide, an isostearyl trimethyl ammonium chloride, a behenyl trimethyl ammonium chloride, a cocoyl trimethyl ammonium chloride, a dodecyl hexadecyl trimethylammoniumchloride, dialkyl (12 to 18) dimethyl ammonium chloride solution, a dicetyl dimethyl ammonium chloride, a distearyl dimethyl ammonium chloride, a dicocoyl dimethyl ammonium chloride, an isostearyl lauryl dimethyl ammonium chloride, a dodecyl dimethyl ethyl ammonium chloride, or the like can be presented.

As the cationic surfactant, it is preferable that one or two of $R^1$ to $R^4$ is a straight chain or branched alkyl group or alkenyl group having 12 to 30 carbon atoms, and the remainder are a methyl group.

The cationic surfactants can be used by two or more kinds in combination, and the content thereof is, from the viewpoints of the necessary and sufficient conditioning effect, the hair damage restraint and the storage stability, preferably 0.1 to 10% by weight in the reducing composition of the present invention, more preferably 0.2 to 7.5% by weight, and even more preferably 0.5 to 5% by weight.

As the polyols of the component (D), alkylene glycols such as an ethylene glycol, a diethylene glycol, a propylene glycol, a dipropylene glycol, a 1, 3-butylene glycol, a hexylene glycol, a triethyleneglycol, and a polyethyleneglycol (molecular weight 200 to 5, 000) ; glycerins such as a glycerin, a diglycerin, and a polyglycerin; saccharides such as a sorbitol, and a sugar alcohol, or the like can be presented. Among these examples, from the viewpoints of obtaining the preferable wave providing effect and the curly hair correcting effect while restraining the damage to the hair, a propylene glycol, a 1,3-butylene glycol, a glycerin, and a polyethylene glycol (molecular weight 200 to 1,000) are preferred. These can be used by two or more kinds in combination, and the content thereof is, from the viewpoints of the hair deforming effect (wave providing effect and curly hair correcting effect), the conditioning effect, the hair damage restraint, and the ease of formulating, preferably 0.1 to 10% by weight in the reducing composition of the present invention, and even more preferably 0.5 to 5% by weight.

As the direct function of the component (D) in the present invention, as the above-mentioned explanation, the hair conditioning effect and the damage restraining effect can be presented, however, since the increase of the use amount of the component (D) leads to the concentration reduction of the water as the diluting agent. As a result, the component (B) is condensed indirectly to bring about troubles such as excessive application of the permanent wave, and the hair damage increases. Considering the optimum quantitative correlation of the component (B) and the component (D) in view of these opposite effects, it is preferable that the multiple of the mole concentration (mol/L) of the former and the weight percentage (% by weight) of the latter is 0.027 to 1.56, more preferably, 0.109 to 1.56, and even more preferably in a range of 0.109 to 1.30 at the same time with the composition range of each of the component (B) and the component (D).

In the reducing composition of the present invention, in addition to the above-mentioned components, for swelling the hair to stably carry out the reducing reaction, a component (E) alkaline agent may be contained. As the alkaline agent, alkanolamines such as a monoethanol amine, a diethanol amine and a triethanol amine, an ammonium, an ammonium hydrogen carbonate, a sodium hydroxide, a potassium hydroxide, or the like can be presented. In the present invention, in the case of having the component (B) in a low concentration while using the component (A) and the component (B) in combination, the characteristics of little stimulating odor can be obtained. In order to utilize the characteristics, it is preferable to use an alkanolamine as the alkaline agent. These alkaline agents may be used by two or more kinds in combination, and furthermore, the content thereof is preferably 0.1 to 10% by weight in the reducing composition of the present invention, and even more preferably in a range of 0.5 to 7.5% by weight in terms of the hair deforming effect (wave providing effect, curly hair correcting effect) and the safety.

Furthermore, in the reducing composition of the present invention, for the purpose of restraining the unnecessary coloring phenomenon and ensuring a stable reducing reaction, a component (F) chelating agent having the function of stably forming a composite substance with a metal ion in the composition can be contained. As the chelating agent, an ethylene diamine tetraacetate and a salt thereof , a hydroxy ethyl ethylene diamine tetraacetate and a salt thereof, a dihydroxy ethyl ethylene diamine tetraacetate and a salt thereof, a 1-hydroxy ethane-1,1-diphosphonic acid and a salt thereof, a nitrilotrismethylene diphosphonic acid and a salt thereof, and a 8-quinolinol and a salt thereof can be presented. These chelating agents may be used by two or more kinds in combination, and the content thereof is in a range of 0.005 to 5% by weight in the total composition, and preferably in a range of 0.01 to 2% by weight in terms of the hair deforming effect (wave effect, curly hair correcting effect), the stability and the safety.

Furthermore, an organic solvent such as an alcohol having 1 to 6 carbon atoms; a higher alcohol, a fatty acid or salts thereof, a cholesterol or a derivative thereof, a vaseline, a lanolin derivative, and an oil based component such as polyethylene glycol aliphatic esters; a natural ceramide or a ceramide analog having a ceramide like function; a moisturizing agent; a chemical such as a vitamin; a protein hydrolyzed product and an amino acid or a derivative thereof; polymer fine powders or a hydrophobic processed product thereof, such as a polyethylene, a polystyrene, a polymethyl methacrylate, a nylon, a silicone, or the like; an extract derived from animals and plants; an ultraviolet ray absorbing agent; a surfactant other than the component (C); a pearly agent; an antiseptic; a disinfectant; an anti-inflammation

agent; a polymer compound such as silicones, modified silicones, a cationic polymer, and an amphoteric polymer; an anti-dandruff agent; a pH adjusting agent; a pigment; a perfume, or the like may be included according to the purpose.

The reducing composition of the present invention can be provided in any type such as a liquid, a gel, an emulsion, a foam (aerosol type, pump foamer type), or the like including the above-mentioned composition used for an ordinary permanent agent with water as a medium.

The reducing composition of the present invention has a pH in a range of 8 to 10 in terms of the hair deforming effect (wave providing effect, curly hair correcting effect), pH 8.25 to 9.75 is preferable, and pH 8.5 to 9.5 is even more preferable.

For carrying out the permanent wave or straight permanent process using the reducing composition of the present invention, the hair can be processed with an oxidizing composition containing a keratin oxidizing substance selected from the group consisting of a bromate and a hydrogen peroxide after processing the hair with the reducing composition of the present invention, washing with water or without washing.

The oxidizing composition used after the reducing process with the reducing composition of the present invention includes a composition used for an ordinary permanent agent with water as a medium, and it can be provided in any type such as a solution, a gel, an emulsion, a foam, or the like.

The oxidizing composition may contain a pH buffer agent for setting pH suitable for each oxidizing agent. As the pH buffer agent, an organic acid used for cosmetics or permanent agents, such as a formic acid, a glycolic acid, an acetic acid, a lactic acid, an oxalic acid, a tartaric acid, a malic acid, a succinic acid, and a citric acid, an inorganic acid such as a phosphoric acid, an alkaline metal salt as a salt thereof, such as a sodium salt and a potassium salt, an alkaline earth metal salt and an ammonium salt can be presented.

At the time of preparing the oxidizing composition as an emulsion, it is preferable to contain , together with a surfactant ordinarily used for this purpose, a straight chain or branched, saturated or unsaturated aliphatic alcohol having 12 to 30 carbon atoms, preferably 12 to 24 carbon atoms, even more preferably 16 to 22 carbon atoms, a straight chain or branched, saturated or unsaturated, fatty acid, which may have a hydroxyl group having 10 to 30 carbon atoms, preferably 12 to 24 carbon atoms, even more preferably 16 to 22 carbon atoms or salts thereof, and a mixture thereof. Moreover, at the time of preparing the oxidizing composition as a gel-like or slightly viscous lotion, it is preferable to contain a cationic, anionic or nonionic water soluble polymer as the viscosity adjusting agent.

Furthermore, in the oxidizing composition, in addition to these contained components, as needed, various cosmetic materials can be included. For example, as a dissolving agent for various composition components, a lower alcohol having 1 to 6 carbon atoms, an ionic (anionic or cationic) surfactant, or a nonionic surfactant may be included. For the purpose of improvement of the feeling upon use, an oil agent, silicones, a cationic polymer, an amphoteric polymer, or the like, for the purpose of the hair damage reduction or the hair protection, an amino acid or a derivative thereof, a protein, hydrolyzed products thereof or a derivative thereof, a natural ceramide or pseudo-ceramides having the similar function can be contained. Furthermore, a perfume providing agent, a coloring agent, an antiseptic, an ultraviolet ray absorbing agent, a moisturizing agent, a chelating agent, a hair growing component, or the like may be contained appropriately.

[Examples]

Example 1, Comparative Examples 1 to 8

Reducing compositions (first agent) shown in Table 1 and an oxidizing composition (second agent) shown in Table 2 were prepared as wave permanent agents for evaluation. Using the wave permanent agents, the permanent process was carried out for evaluating the hair loss modulus, the permanent effect, and the hair bundle feeling after permanent application by the following methods.

<Permanent process>

By preliminarily carrying out a bleaching process (process by 10 times with INAZUMA BLEACH produced by Kao Corporation, 1:1 bath ratio, and left at room temperature for 20 minutes), a drastically damaged hair bundle of 0.1 g, 20 cm length was produced. This was wound around spirally onto a cylindrical glass rod having a diameter of 15 mm so as to have the first agent applied by a 1:1 bath ratio (hair : agent) and spread evenly, and left at room temperature for 15 minutes. Then, the hair bundle wound around the rod was rinsed with running water having a temperature of 40°C for 30 seconds. After removing the water with a towel, the second agent was applied by the 1:1 bath ratio, spread evenly, and it was left at room temperature for 15 minutes. Finally, the hair bundle was detached from the rod and rinsed with running water having a temperature of 40°C for 30 seconds so as to finish the wave permanent process.

<Evaluation>

- Measurement of modulus of elasticity

A measurement kit shown in FIG. 1 was produced by suspending a hair specimen having a length of 2 cm by fixing one end thereof to the center of gravity portion of a glass having a diameter of 3 mm, a length of 5 cm, fixing the other end thereof above with a vinyl tape or the like. Using the measurement kit, the loss modulus by the permanent process was measured.

When the glass rod balanced horizontally is twisted and rotated around the portion with the hair sample fixed, the cycle T (second) depends on the hardness of the hair (formula 1). Here, I denotes the inertial moment of the glass rod, and C the twist constant of the hair sample. Moreover, the value obtained by dividing the twist constant C by the 4th power of the diameter d of the hair sample is proportional to the twist elastic modulus $\gamma$ of the hair (formula 2). With the premise that the twist elastic modulus before the permanent process is $\gamma_0$ and the twist elastic modulus of the hair in an optional state during the permanent process is $\gamma_i$, the ratio can be calculated as the ratio of the square of the rotation cycle ($T_0$, $T_i$) of the glass rod measured in the unprocessed state and the optional state (formula 3).

$$C = 4\pi^2 I / T^2 \qquad \text{(formula 1)}$$

$$\gamma \propto C / d^4 \qquad \text{(formula 2)}$$

$$\gamma_i / \gamma_0 = T_0^2 / T_i^2 \qquad \text{(formula 3)}$$

Therefore, with the twist elasticity before the permanent provided as 100%, by measuring the rotation cycle of the glass rod immediately after the reducing process with the first agent and immediately after the oxidizing process with the second agent, the twist elastic modulus immediately after the reduction and immediately after the oxidization can be found so that the loss modulus can be determined from the difference thereof.

In order to obtain a sufficient wave providing effect, it is necessary to sufficiently lose the elasticity of the hair by the reducing process with the first agent as well as to restore the same by the oxidizing process with the second agent as much as possible. That is, if the loss modulus by the first agent process is large and the loss modulus through the entire process is small, a desired shape can be provided while restraining loss of the elasticity to the minimum level.

Table 1 shows the loss modulus, with the loss modulus of less than 30% shown as A, and the loss modulus of 30% or more as B. Since the loss modulus of 30% or more leads to the generation of cut hair and split ends, it is not preferable.

- Measurement of the curl degree and the wave degree

With the premise that the diameter of the glass rod used for the permanent process is $A_0$ (= 15 mm), the length of the original hair bundle is $L_0$ (= 20 cm), after permanent process, the diameter of the curl formed in a state with the hair bundle wet with water is A (mm), and the length of the hair bundle is L (cm), the curl degree and the wave degree were calculated from the following formulae. The length of the hair was measured in a state with the base of the hair bundle fixed and suspended vertically.

Table 1 shows the curl degree and the wave degree, with the case with the preferable effect of the 35 to 50% curl degree and the 15 to 30% wave degree shown as A, and the case outside the criteria shown as B. In the case of less than the above-mentioned criteria, the permanent effect is too weak, and in the case of more than the above-mentioned criteria, the permanent is applied excessively, so that it is not preferable in terms of the esthetic viewpoint.

$$\text{Curl degree (\%)} = (A_0/A) \times 100$$

$$\text{Wave degree (\%)} = [(L_0 - L) \div L_0] \times 100$$

-Feeling evaluation of the hair bundle after the permanent process

As to the tress with the permanent process applied by the above-mentioned method, feeling evaluation was carried out according to the following evaluation criteria by specialist panelists (5 persons). Table 1 shows the evaluation average values, with the average value of 3 or more shown as "A", and the average value of less than 3 shown as "B" because the 3 or more evaluation is a use feeling tolerable in terms of the use.

[Evaluation criteria]

4: The hair feels soft and flexible.
3: The hair slightly feels soft and flexible.
2: The hair feels rather dry and rough.
1: The hair feels dry and rough.

[Table 1]

| First agent (% by weight, mol/L in the parentheses) | | Example 1 | Comparative Example 1 | Comparative Example 2 | Comparative Example 3 | Comparative Example 4 | Comparative Example 5 | Comparative Example 6 | Comparative Example 7 | Comparative Example 8 |
|---|---|---|---|---|---|---|---|---|---|---|
| (A) | Sodium sulfite | 4.5 (0.36) | 4.5 (0.36) | - | 4.5 (0.36) | 4.5 (0.36) | 4.5 (0.36) | 8.2 (0.65) | 0.1 (0.01) | 4.5 (0.36) |
| (B) | Monoethanol amine thioglycolate solution (containing 50% by weight of a thioglycolic acid) | 1.5 (0.08) | - | 1.5 (0.08) | 1.5 (0.08) | 1.5 (0.08) | 1.5 (0.08) | 11.94 (0.65) | 1.5 (0.08) | 0.1 (0.005) |
| (C) | Cetyl trimethyl ammonium chloride (30%) | 2.8 | - | - | - | 2.8 | - | 2.8 | 2.8 | 2.8 |
| (D) | Propylene glycol | 3.2 | - | - | - | - | 3.2 | 3.2 | 3.2 | 3.2 |
| Others | Monoethanol amine | 3.0 | 3.0 | 3.0 | 3.0 | 3.0 | 3.0 | 3.0 | 3.0 | 3.0 |
| | Polyoxy ethylene lauryl ether (23E. O) | 0.9 | 0.9 | 0.9 | 0.9 | 0.9 | 0.9 | 0.9 | 0.9 | 0.9 |
| | Polyoxy ethylene dodecyl ether (9E. O) | 0.6 | 0.6 | 0.6 | 0.6 | 0.6 | 0.6 | 0.6 | 0.6 | 0.6 |
| | Disodium edentate | 0.5 | 0.5 | 0.5 | 0.5 | 0.5 | 0.5 | 0.5 | 0.5 | 0.5 |
| | Methyl paraoxybenzoate | 0.1 | 0.1 | 0.1 | 0.1 | 0.1 | 0.1 | 0.1 | 0.1 | 0.1 |
| | Citric acid | Optional amount | Optional amount | Optional amount | Optional amount | Optional amount | Optional amount | Optional amount | Optional amount | Optional amount |
| | Refined water | Remainder | Remain der | Remain der | Remainder | Remainder | Remainder | Remainder | Remainder | Remainder |

(continued)

| First agent | Example | Comparative Examples | | | | | | | |
|---|---|---|---|---|---|---|---|---|---|
| (% by weight, mol/L in the parentheses) | 1 | 1 | 2 | 3 | 4 | 5 | 6 | 7 | 8 |
| pH | 9.2 | 9.2 | 9.2 | 9.2 | 9.2 | 9.2 | 9.2 | 9.2 | 9.2 |
| Loss modulus by the first agent $\gamma_{i1}$ (%) | 45 | 10 | 57 | 50 | 59 | 61 | 87 | 52 | 19 |
| Restored elastic modulus by the second agent $\gamma_{i2}$ (%) | 21 | 2 | 24 | 21 | 24 | 22 | 15 | 16 | 4 |
| $\gamma_{i1}$-$\gamma_{i2}$ = Loss modulus by the permanent(%) | 24 | 8 | 33 | 29 | 35 | 39 | 72 | 36 | 15 |
| (less than 30% loss modulus) | (A) | (A) | (B) | (A) | (B) | (B) | (B) | (B) | (A) |
| Curl degree (%) | 46.9 | 37.5 | 41.7 | 44.1 | 43.9 | 46.5 | 54.2 | 47.4 | 45.4 |
| (35 to 50%) | (A) | (A) | (A) | (A) | (A) | (A) | (B) | (A) | (A) |
| Wave degree (%) | 24.5 | 14.5 | 16.2 | 12.2 | 15.4 | 15.8 | 26.6 | 14.6 | 13.0 |
| (15 to 30%) | (A) | (B) | (A) | (B) | (A) | (A) | (A) | (B) | (B) |
| Feeling of the hair bundle after the permanent | 3.8 | 2.8 | 2.6 | 2.4 | 3.4 | 2.6 | 1.6 | 3.8 | 3.8 |
| (4: good to 1: poor) (Average 3 or more) | (A) | (B) | (B) | (B) | (A) | (B) | (B) | (A) | (A) |

[Table 2]

| (% by weight) | second agent |
|---|---|
| Sodium bromate | 8.0 |
| Propylene glycol | 1.0 |
| Stearyl trimethyl ammonium chloride (28% by weight) | 1.0 |
| Polyoxy ethylene lauryl ether (23E.O.) | 0.6 |
| Polyoxy ethylene dodecyl ether (9E.O.) | 0.4 |
| Disodium edentate | 0.5 |
| Citric acid | 0.5 |
| Sodium hydroxide | Optional amount |
| Cationized cellulose (Kao Corporation, KACHISERO L150) | 0.1 |
| Refined water | Remainder |
| pH | 6.0 |

From the above-mentioned results, it is learned that the reducing composition of Example 1 can provide the desired shape while restraining the hair damage such as loss of elasticity and deterioration of feel to the touch without lowering the wave provision or sustenance even to the drastically damaged hair.
The permanent process was carried out by the same method as in Example 1 using the reducing compositions of Examples 2 to 5 and the oxidizing composition of Table 2 shown below. As a result, it was confirmed that all of them have the excellent wave providing effect with preferable hair feel to the touch and little damage in terms of the hair elasticity.

Examples 2 to 5 and Comparative Examples 9 to 10

By preparing 6 kinds of the permanent first agents with only the composition amount of the component (B) changed as shown in Table 3, the degree of the damage provided to the hair thereby was compared.

(Experimental method)

In general, hair shrinks with the damage proceeding. At the level of generating the excessive shrinkage (drastic shrinkage), about 2% or more shrinkage is generated. Utilizing this nature, the permanent damage at the time of applying the permanent process to the drastically damaged hair was evaluated.
By preliminarily carrying out a bleaching process (process by 10 times with INAZUMA BLEACH produced by Kao Corporation, 1 : 1 bath ratio, left at room temperature for 20 minutes), using 10 pieces of the drastically damaged hair, a 17 cm length small hair bundle (Japanese hair) with one end of the hair fixed with an adhesive tape was produced. The small hair bundle was embedded in a 0.1 g hair bundle so as to have the permanent process for precisely controlling the bath ratio of the permanent agent. Moreover, since an error is generated due to the difference of the tension at the time of winding if the permanent process is carried out with the hair wound around a rod at the time of the shrinkageratiomeasurement, the process with the permanent agent was carried out without winding around a rod. The damage degree was evaluated from the shrinkage degree of the small hair bundle at the time of applying the permanent process three times to the hair bundle.
For the permanent process, the first agent shown in Table 3 was applied to the hair by the 1:1 bath ratio, and left at room temperature for 15 minutes. Then, it was rinsed with hot water for 30 seconds. After removing the water with a towel, the second agent (8% by weight aqueous solution of a sodium bromate) was applied by the 1:1 bath ratio. After leaving at room temperature for 15 minutes, it was rinsed out with hot water, and dried naturally so as to finish the process. The length of the small hair bundle was measured before and after the permanent process for calculating the hair shrinkage ratio due to the permanent process from the following formula. The shrinkage ratio of less than 1.5% was evaluated as the range with the damage due to the permanent process sufficiently restrained (the shrinkage ratio of less than 1.5% is shown as A, and 1.5% or more as B, in Table 3). The length of the small hair bundle was measured in a state with one end pulled lightly with a hand for eliminating the slack.
$$\text{Shrinkage ratio (\%)} = L/L_0 \times 100$$

$L_0$: length of the hair before the permanent process (17 cm)
L: length of the hair after carrying out the permanent process for 3 times (cm)

[Table 3]

| (% by weight, mol/L in the parentheses) | | Examples | | | | Comparative Examples | |
|---|---|---|---|---|---|---|---|
| | | 2 | 3 | 4 | 5 | 9 | 10 |
| (A) | Sodium sulfite | 3.0 (0.24) | ← | ← | ← | ← | |
| (B) | Monoethanolamine thioglycolate solution (containing 50% by weight of a thioglycolic acid) | 2.0 (0.11) | 2.4 (0.13) | 2.8 (0.15) | 3.2 (0.17) | 5.0 (0.27) | 6.0 (0.33) |
| (C) | Cetyl trimethyl ammonium chloride solution (30% by weight) | 3.08 | ← | ← | ← | ← | ← |
| (D) | Propylene glycol | 3.2 | ← | ← | ← | ← | ← |
| Others | Monoethanolamine | 3.0 | ← | ← | ← | ← | ← |
| | Isostearyl glyceryl ether | 0.1 | ← | ← | ← | ← | ← |
| | Polyoxy ethylene lauryl ether (23E. O.) | 0.99 | ← | ← | ← | ← | ← |
| | Polyoxy ethylene dodecyl ether (9E.O.) | 0.66 | ← | ← | ← | ← | ← |
| | Amino modified silicone (SM8704C, Dow Corning Toray Silicone Co., Ltd) | 1.0 | ← | ← | ← | ← | ← |
| | Disodium edentate | 0.5 | ← | ← | ← | ← | ← |
| | Methyl paraoxybenzoate | 0.25 | ← | ← | ← | ← | ← |
| | Perfume | Optional amount | ← | ← | ← | ← | ← |
| | 50% by weight Citric acid | pH9.2 | ← | ← | ← | ← | ← |
| | Ion exchange water | Remain der | ← | ← | ← | ← | ← |
| Hair damage after the permanent Hair shrinkage ratio after the permanent (%) (less than 1.5 % hair shrinkage ratio) | | 0<br>(A) | 0.04<br>(A) | 0.11<br>(A) | 0.54<br>(A) | 1.60<br>(B) | 3.29<br>(B) |

Examples 6 to 8 and Comparative Example 11

By preparing 4 kinds of the permanent first agents with only the composition amount of the component (D) changed as shown in Table 4, and carrying out the same permanent process to the same hair bundle as in Examples 2 to 5 and Comparative Examples 9 to 10, the degree of the damage provided to the hair was compared.
Furthermore, the same permanent process was carried out to the same hair bundles of Example 1 and Comparative Examples 1 to 8, and the curl degree and the wave degree were calculated from the above-mentioned formulae for the hair after the permanent process.
[Table 4]

| (% by weight, mol/L in the parentheses) | | Examples | | | Comparative Example |
|---|---|---|---|---|---|
| | | 6 | 7 | 8 | 11 |
| (A) | Sodium sulfite | 3.0 (0.24) | ← | ← | ← |
| (B) | Monoethanolamine thioglycolate solution (containing 50% by weight of a thioglycolic acid) | 2.4 (0.13) | ← | ← | ← |
| (C) | Cetyl trimethyl ammonium chloride solution (30% by weight) | 3.08 | ← | ← | ← |
| (D) | Propylene glycol | 3.2 | 6.4 | 9.6 | 12.8 |
| Others | Monoethanolamine | 3.0 | ← | ← | ← |
| | Isostearyl glyceryl ether | 0.1 | ← | ← | ← |
| | Polyoxy ethylene lauryl ether (23E. O.) | 0.99 | ← | ← | ← |
| | Polyoxyethylene dodecyl ether (9E. O.) | 0.66 | ← | ← | ← |
| | Amino modified silicone (SM8704C, Dow Corning Toray Silicone Co., Ltd) | 1.0 | ← | ← | ← |
| | Disodium edentate | 0.5 | ← | ← | ← |
| | Methyl paraoxybenzoate | 0.25 | ← | ← | ← |
| | Perfume | Optional amount | ← | ← | ← |
| | 50% by weight Citric acid | pH9.2 | ← | ← | ← |
| | Ion exchange water | Remainder | ← | ← | ← |
| Hair damage after the permanent<br>Hair shrinkage ratio after the permanent (%)<br>(less than 1.5% hair shrinkage ratio is A, 1.5% or more isB) | | 0.15<br>(A) | 0.16<br>(A) | 0.24<br>(A) | 1.35<br>(A) |
| Curl degree (%)<br>(less than 50% is A, 50% or more is B) | | 46.2<br>(A) | 47.4<br>(A) | 49.2<br>(A) | 50.9<br>(B) |
| Wave degree (%)<br>(less than 30% is A, 30% or more is B) | | 24.5<br>(A) | 21.7<br>(A) | 23.6<br>(A) | 34.0<br>(B) |

Examples 9 to 11

By preparing 3 kinds of the permanent first agents with the composition amount of the component (D) changed

as shown in Table 5, and carrying out the same permanent process to the same hair bundle as in Examples 2 to 5 and Comparative Examples 9 to 10, the degree of the damage provided to the hair was compared.

[Table 5]

| (% by weight, mol/L in the parentheses) | | Examples | | |
|---|---|---|---|---|
| | | 9 | 10 | 11 |
| (A) | Sodium sulfite | 3<br>(0.24) | ← | ← |
| (B) | Monoethanolamine thioglycolate solution (containing 50% by weight of a thioglycolic acid) | 3.2<br>(0.17) | ← | ← |
| (C) | Cetyl trimethyl ammonium chloride solution (30% by weight) | 3.08 | ← | ← |
| (D) | Propylene glycol | 3.2 | 6.4 | 9.6 |
| Others | Monoethanolamine | 3.0 | ← | ← |
| | Isostearyl glyceryl ether | 0.1 | ← | ← |
| | Polyoxy ethylene lauryl ether (23E. O.) | 0.99 | ← | ← |
| | Polyoxy ethylene dodecyl ether (9E. O.) | 0.66 | ← | ← |
| | Amino modified silicone (SM8704C, Dow Corning Toray Silicone Co., Ltd) | 1.0 | ← | ← |
| | Disodium edentate | 0.5 | ← | ← |
| | Methyl paraoxybenzoate | 0.25 | ← | ← |
| | Perfume | Optional amount | ← | ← |
| | 50 % by weight Citric acid | pH9.2 | ← | ← |
| | Ion exchange water | Remainder | ← | ← |
| Mole concentration of (B) (mol/L) x weight percentage of (D) (% by weight) | | 0.56 | 1.11 | 1.67 |
| Hair damage after the permanent<br>Hair shrinkage ratio after the permanent (%)<br>(less than 1.5% hair shrinkage ratio is A, 1.5% or more is B) | | 0<br>(A) | 0.36<br>(A) | 1.19<br>(A) |

Example 12

| | (% by weight) |
|---|---|
| Sodium sulfite | 3.5 (0.28 mol/L) |
| Monoethanolamine thioglycolate solution (containing 50% by weight of a thioglycolic acid) | 2.0 (0.11 mol/L) |
| Monoethanol amine | 3.0 |
| Propylene glycol | 3.2 |
| Cetyl trimethyl ammonium chloride solution(30% by weight) | 2.8 |
| Polyoxy ethylene lauryl ether (23E.O.) | 0.9 |
| Polyoxy ethylene dodecyl ether (9E.O) | 0.6 |
| Amino modified silicone*1 | 1.0 |
| Isostearyl glyceryl ether | 0.1 |
| Trimethyl glycine | 0.25 |
| Disodium edentate | 0.5 |
| Methyl paraoxybenzoate | 0.1 |

(continued)

| | (% by weight) |
|---|---|
| Citric acid | optional amount |
| Refined water | balance |
| Perfume | Optional amount |
| pH 9.2 | total) 100% |
| *1 SM8704C, Dow Corning Toray Silicone Co., Ltd | |

Example 13

| | (% by weight) |
|---|---|
| Sodium hydrogen sulfite | 3.0 (0.24 mol/L) |
| L-cysteine | 1.0 (0.08 mol/L) |
| Monoethanolamine | 3.0 |
| Propylene glycol | 3.2 |
| Stearyl trimethyl ammonium chloride solution (30% by weight) | 2.8 |
| Polyoxy ethylene lauryl ether (23E.O.) | 0.9 |
| Polyoxy ethylene dodecyl ether (9E.O) | 0.6 |
| Amino modified silicone *1 | 1.0 |
| Isostearyl pentaerythryl glyceryl ether | 0.2 |
| Isostearyl glyceryl ether | 0.1 |
| Trimethyl glycine | 0.25 |
| Disodium edentate | 0.5 |
| Methyl paraoxybenzoate | 0.1 |
| Citric acid | Optional amount |
| Refined water | balance |
| Perfume | Optional amount |
| pH 9.2 | Total) 100% |
| *1 SM8704C, Dow Corning Toray Silicone Co., Ltd | |

Example 14

| | (% by weight) |
|---|---|
| Sodium sulfite | 2.0 (0.17 mol/L) |
| Thiolactic acid | 1.0 (0.09 mol/L) |
| Monoethanolamine | 3.0 |
| Propylene glycol | 3.2 |
| Cetyl trimethyl ammonium chloride solution (30% by weight) | 2.8 |
| Polyoxy ethylene lauryl ether (23E.O.) | 0.9 |
| Polyoxy ethylene dodecyl ether (9E.O) | 0.6 |
| High polymerization methyl polysiloxane emulsion (T60)*1 | 0.5 |
| Dimethyl diallyl ammonium acrylamide copolymer solution*2 | 0.1 |
| Trimethyl glycine | 0.25 |
| Disodium edentate | 0.5 |
| Methyl paraoxybenzoate | 0.1 |
| Citric acid | optional amount |

(continued)

| | (% by weight) |
|---|---|
| Refined water | balance |
| Perfume | Optional amount |
| pH 9.2 | total) 100% |
| *1 Silicone BY22-060, Dow Corning Toray Silicone Co., Ltd<br>*2 MERQUAT 550, ONDEO NALCO Corp. | |

Example 15

| | (% by weight) |
|---|---|
| Sodium sulfite | 3.0 (0.23 mol/L) |
| Monoethanolamine thioglycolate solution (containing 50% by weight of a thioglycolic acid) | 2.0 (0.11 mol/L) |
| Monoethanolamine | 3.0 |
| Glycerin | 3.2 |
| Cetyl trimethyl ammonium chloride solution (30% by weight) | 2.8 |
| Polyoxy ethylene lauryl ether (23E.O.) | 0.9 |
| Polyoxy ethylene dodecyl ether (9E.O) | 0.6 |
| Amino modified silicone *1 | 1.0 |
| Long chain dibasic acid bis 3-methoxy propylamide | 0.1 |
| Trimethyl glycine | 0.2 |
| Disodium edetate | 0.5 |
| Methyl paraoxybenzoate | 0.1 |
| Citric acid | optional amount |
| Refined water | balance |
| Perfume | Optional amount |
| pH 9.2 | total) 100% |
| *1 SM8704C, Dow Corning Toray Silicone Co., Ltd | |

**Claims**

**1.** A reducing composition for permanent wave or straight permanent having a composition pH in a range of 8 to 10 , comprising the following components (A) to (D):

(A) 0.039 to 0.39 mol/L of a keratin reducing substance not having a mercapto group selected from the group consisting of a sulfite, ahydrogensulfite, apyrosulfiteandathiosulfate,
(B) 0.007 to 0.196 mol/L of a keratin reducing substance having a mercapto group selected from the group consisting of a thioglycol acid and a salt thereof, a cysteine and a salt thereof, an acetyl cysteine, a cysteamine and a salt thereof, and a thiolactic acid and a salt thereof,
(C) 0.1 to 10% by weight of a cationic surfactant represented by the following general formula:

[chemical formula 1]

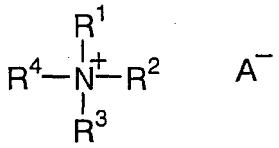

wherein one or two of $R^1$, $R^2$, $R^3$ and $R^4$ is a hydrocarbon group having 12 to 30 carbon atoms, which may have a substituent, and the remainder are a hydrocarbon group having 1 to 3 carbon atoms, which may have a substituent, and $A^-$ is an anion, and
(D) 0.1 to 10% by weight of polyols.

**2.** The reducing composition according to claim 1, comprising the component (A) by 0.039 to 0.31 mol/L, the component (B) by 0.007 to 0.196 mol/L, the component (C) by 0.2 to 7.5% by weight, and the component (D) by 0.5 to 5% by weight.

**3.** The reducing composition according to claim 1, comprising the component (A) by 0.039 to 0.31 mol/L, the component (B) by 0.007 to 0.163 mol/L, the component (C) by 0.2 to 7.5% by weight, and the component (D) by 0.5 to 5% by weight.

**4.** The reducing composition according to claim 1, comprising the component (A) by 0.039 to 0.31 mol/L, the component (B) by 0.007 to 0. 098 mol/L, the component (C) by 0.2 to 7.5% by weight, and the component (D) by 0.5 to 5% by weight.

**5.** The reducing composition according to any of claims 1 to 4, wherein the product obtained by multiplying the mole concentration (mol/L) of the component (B) by the weight percentage (% by weight) of the component (D) is in a range of 0.027 to 1.56.

**6.** The reducing composition according to any of claims 1 to 5, further comprising a component (E) alkaline agent, a component (F) chelating agent and a component (G) water.

**7.** The reducing composition according to claim 6, comprising the component (E) by 0.1 to 10% by weight, and the component (F) by 0.005 to 5% by weight.

**8.** A permanent wave or straight permanent processing method of processing hair with an oxidizing composition comprising a keratin oxidizing substance selected from the group consisting of a bromate and a hydrogen peroxide after processing the hair with the reducing composition according to any of claims 1 to 7, washing with water or without washing.

Fig. 1

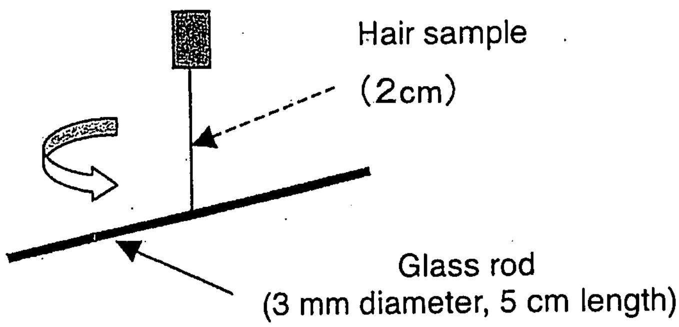

**INTERNATIONAL SEARCH REPORT**

International application No.
PCT/JP2005/012692

A. CLASSIFICATION OF SUBJECT MATTER
***A61K8/23*** (2006.01), ***A61K8/46*** (2006.01), ***A61K8/40*** (2006.01), ***A61K8/34*** (2006.01), ***A61Q5/04*** (2006.01)

According to International Patent Classification (IPC) or to both national classification and IPC

B. FIELDS SEARCHED

Minimum documentation searched (classification system followed by classification symbols)
***A61K8/23*** (2006.01), ***A61K8/46*** (2006.01), ***A61K8/40*** (2006.01), ***A61K8/34*** (2006.01), ***A61Q5/04*** (2006.01)

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 63-190814 A (The Gillette Co.), 08 August, 1988 (08.08.88), Claims; page 5, lower left column, line 6 to lower right column, line 14; examples & US 5294230 A & EP 257256 A2 & DE 3781626 A | 1-8 |
| Y | JP 63-188618 A (Shiseido Co., Ltd.), 04 August, 1988 (04.08.88), Claims; page 3, upper right column, lines 1 to 10; example 3 (Family: none) | 1-8 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>18 October, 2005 (18.10.05) | Date of mailing of the international search report<br>01 November, 2005 (01.11.05) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2005/012692

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2-73006 A (Shiseido Co., Ltd.), 13 March, 1990 (13.03.90), Claims; page 4, upper right column, lines 8 to 9; example 6 (Family: none) | 1-8 |
| Y | JP 2002-187822 A (Kao Corp.), 05 July, 2002 (05.07.02), Claims; Par. Nos. [0014], [0027], [0031], [0036] (Family: none) | 1-8 |
| Y | JP 2003-40741 A (Hoyu Co., Ltd.), 13 February, 2003 (13.02.03), Scope of claims; Claims 6 to 7 Par. Nos. [0032], [0058] (Family: none) | 1-8 |
| Y | JP 8-325123 A (Hoyu Co., Ltd.), 10 December, 1996 (10.12.96), Claims; Par. Nos. [0003], [0008]; table 5; Par. Nos. [0030] to [0031] (Family: none) | 1-8 |
| Y | JP 10-337211 A (Wella AG.), 22 December, 1998 (22.12.98), Claims; Par. Nos. [0004] to [0008] & EP 880916 A1 | 1-8 |
| A | JP 60-1114 A (Lion Corp.), 07 January, 1985 (07.01.85), Claims; page 1, right column to page 2, upper left column (Family: none) | 1-8 |
| A | JP 51-29235 A (Shiseido Co., Ltd.), 12 March, 1976 (12.03.76), Claims; page 1, left column, 3rd line from the bottom to right column (Family: none) | 1-8 |
| A | JP 6-192052 A (Wella AG.), 12 July, 1994 (12.07.94), Claims; Par. Nos. [0003] to [0004] & DE 4240471 A1 & EP 604717 A2 | 1-8 |
| A | JP 10-87447 A (Kao Corp.), 07 April, 1998 (07.04.98), Claims; Par. Nos. [0003] to [0004] & DE 19633112 A1 | 1-8 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 57002216 A
- JP 63190814 A
- JP 2138112 A